# FASCICULE DE BREVET EUROPEEN

(11) **EP 0 546 897 B1**
(45) Date de publication et mention de la délivrance du brevet: **31.01.1996**
(21) Numéro de dépôt: 92403272.5
(22) Date de dépôt: 03.12.1992
(51) Int. Cl.: C08J 9/10

(54) **Agents moussants des polymères et leur application à la préparation de mousses synthétiques**
Treibmittel für Polymere und ihre Anwendung für die Herstellung synthetischer Schäume
Blowing agents for polymers and their use in the preparation of synthetic foams

(30) Priorité: 12.12.1991 FR 9115427
(43) Date de publication de la demande: 16.06.1993
(73) Titulaire: CRAY VALLEY SA, F-92800 Puteaux (FR)
(72) Inventeur: Bedel, Didier, F-60610 La Croix Saint Ouen (FR); Crozat, Chantal, F-02840 Athies sous Laon (FR)
(74) Mandataire: Dubost, Thierry Pierre

(56) Documents cités:
- Aucun document pertinent relevé

## Description

La présente invention se rapporte à de nouveaux agents moussants des polymères et à leur application à l'obtention de mousses synthétiques dont la fabrication et/ou l'utilisation ne nuit pas à la couche d'ozone de l'atmosphère.

Les mousses phénol-aldéhyde, et plus particulièrement les mousses formo-phénoliques, trouvent actuellement des usages accrus dans le domaine de la construction et dans l'industrie des transports en raison de leur isolation thermique et de leur résistance au feu élevées. Elles sont également utilisées sous forme de blocs à usage domestique tels que des mousses pique-fleurs.

D'autres polymères synthétiques trouvent des applications importantes en volume sous forme de mousses, tels que par exemple les polyuréthanes, le polystyrène, les copolymères styrène-anhydride maléique, le poly-isoprène, le polybutadiène, le polychlorure de vinyle, les copolymères chlorure de vinyle/acétate de vinyle, chlorure de vinyle/éthylène, chlorure de vinyle/chlorure de vinylidiène et chlorure de vinyle/acrylonitrile, le polyéthylène, le polypropylène, les copolymères tétrafluoroéthyléne/hexafluoropropylène, les copolymères éthylène/acétate de vinyle et éthylène/acrylate de butyle, les copolymères greffés de chlorure de vinyle et d'acrylate d'alkyle, les polyamides et les polylactames.

Ces mousses polymères, comme les mousses phénol-aldéhyde, ont en commun le fait que leur fabrication fait généralement appel, en tant qu'agent d'expansion, à un ou plusieurs hydrocarbures, totalement halogénés, notamment les chlorofluorocarbures dits CFC. A l'heure actuelle, l'emploi de chlorofluorocarbones (CFC) pose de graves problèmes écologiques. Ces produits sont en effet soupçonnés de détruire la couche d'ozone stratosphérique, ce qui peut entraîner une pénétration plus importante des rayons UV solaires et, en conséquence, des troubles physiologiques importants chez les êtres vivants.

Les CFC sont utilisés en particulier pour la fabrication de mousses synthétiques. Le remplacement des CFC par un gaz moins nocif représente un progrès écologique important. La solution la plus couramment adoptée à ce jour consiste à remplacer les CFC par des hydrochlorofluorocarbones (HCFC). Ces produits ont été délibérément choisis pour leur plus grande fragilité, de façon à pouvoir se décomposer avant d'atteindre la stratosphère. Toutefois, l'impact environnemental de ces composés reste jusqu'à présent inconnu.

Le problème que la présente invention vise à résoudre consiste à proposer un moyen simple, économique et efficace de protéger la couche d'ozone de l'atmosphère à l'occasion de la fabrication et de l'utilisation de mousses polymères. Dans le contexte plus particulier des mousses phénol-aldéhyde, un second problème que la présente invention vise à résoudre consiste à mettre au point une formulation capable de remplir totalement le moule utilisé à l'occasion de cette fabrication et capable de s'expanser pendant une durée courte tout en conservant les bonnes propriétés d'isolation thermique et acoustique traditionnelles chez les mousses phénol-aldéhyde.

Ces buts sont atteints grâce à la présente invention au moyen d'une composition capable d'être transformée en mousse comprenant un mélange à base de résine polymère et une quantité efficace, par rapport à ladite résine, d'agent d'expansion, ladite composition étant caractérisée en ce que l'agent d'expansion comprend au moins un azoture d'hydrocarbone α, β hydroxylé. Par azoture d'hydrocarbone, α, β hydroxylé au sens de la présente invention, on entend de préférence un composé de formule générale 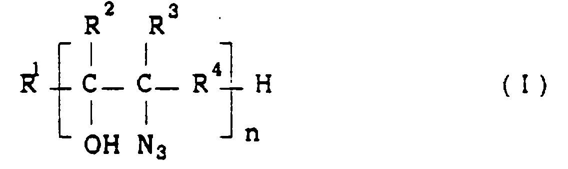 dans laquelle :
- R¹, R et R³, identiques ou différents, sont choisis parmi l'atome d'hydrogène et les radicaux alkyle, aryle, aralkyle, alkylaryle et cycloalkyle ayant de préférence jusqu'à 20 atomes de carbone.
- R⁴ est choisi parmi les groupements alkylènes et arylènes ayant de préférence jusqu'à 20 atomes de carbone, et
- n est un nombre entier compris entre 1 et 20 environ.

La synthèse de tels composés a déjà été décrite dans la littérature, par exemple par addition de nitrure de sodium sur un oxiranne de formule 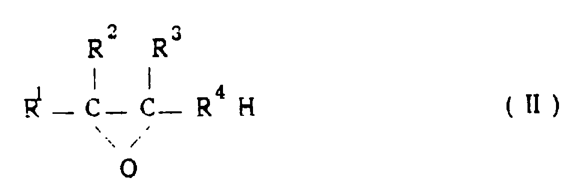 dans laquelle R¹, R, R³ et R⁴ ont les significations indiquées pour la formule (I). Une autre méthode de synthèse connue d'après la littérature consiste en l'addition de nitrure de sodium sur le composé de formule C₆H₅ -- CO -- CH₂ Br, suivie d'une étape de réduction pour obtenir le 2-azido 1-phényléthanol de formule C₆ H₅ -- CHOH -- CH₂ N₃.

Comme exemples d'azotures d'hydrocarbones α, β hydroxyiés utilisables dans la présente invention, on peut citer les composés suivants :
- 2-azido éthanol
- 2-azido 1-phényl éthanol
- 1-azido 2-phényl éthanol
- 1-chloro 3- azido 2 propanol
- 3-chloro 2-azido propanol
- 1-azido 2-butanol
- 1,3-diazido-2-propanol
- 2-azido butanol
- 3-phénoxy 2-azido propanol
- 3 phénoxy 1-azido 2-propanol

Ces composés présentent la particularité d'une parfaite solubilité dans les résols et les polyols et, de manière plus générale, d'une solubilité importante dans les résines polymères hydrophiles. Cette particularité leur procure un avantage important, au surplus du respect de l'environnement, par rapport aux hydrocarbures totalement ou partiellement halogénés tels que les chlorofluorocarbones (CFC) et les chlorotluorocarbones hydrogénés (HCFC), lesquels sont maintenus en émulsion dans les résines hydrophiles généralement par la présence additionnelle d'un surfactant dans la composition moussable. De manière concrète, la solubilité de l'azoture d'hydrocarbone α, β hydroxylé dans la résine polymère évite qu'il ne se dépose lors d'un stockage prolongé de la composition moussable.

Les azotures d'hydrocarbones α, β hydroxylés sont susceptibles, dans les conditions généralement usitées pour le moussage des résines polymères, de se réarranger en libérant de l'azote et en formant une amine secondaire de formule générale : 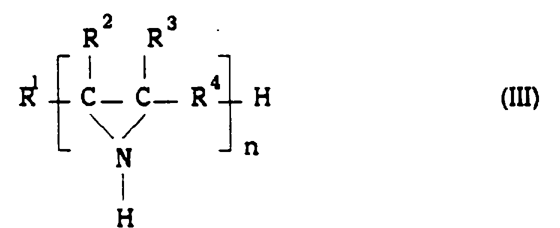 dans laquelle R¹, R, R³ et R⁴ ont les significations indiquées pour la formule (I). Cette amine secondaire est elle-même susceptible de réagir avec la résine polymère, notamment lorsque celle-ci est un résol phénolique ou une résine polyuréthane, ce qui a pour effet de greffer l'agent d'expansion sur le réseau polymère et par conséquent d'empêcher ou tout au moins de limiter considérablement l'extraction de l'agent d'expamion.

La résine polymère présente dans la composition selon l'invention peut être tout polymère de polyaddition ou de polycondensation. thermoplastique ou thermodurcissable, capable d'être expansé. Elle peut être notamment choisie parmi les polyuréthanes, le polystyrène, les copolymères styrène-anhydride maléique, le poly-isoprène, le polybutadiène, le polychlorure de vinyle, les copolymères chlorure de vinyle/acétate de vinyle, chlorure de vinyle/éthylène, chlorure de vinyle/chlorure de vinylidiène et chlorure de vinyle/acrylonitrile, le polyéthylène, le polypropylène, les copolymères tétrafluoroéthylène/hexafluoropropylène, les copolymères éthylène/acétate de vinyle et éthylène/acrylate de butyle, les copolymères greffés de chlorure de vinyle et d'acrylate d'alkyle, les polyamides et les polylactames, ainsi que parmi les résines phénol-aldéhyde. Ces dernières sont des résines bien connues obtenues de préférence par condensation, en milieu basique, d'un aldéhyde tel que le furfural ou le formol et d'un phénol dans un rapport molaire aldéhyde/phénol généralement compris entre 0,8 et 1,5 environ.

Comme indiqué précédemment, la composition selon l'invention doit comprendre une quantité efficace du composé de formule (I), c'est-à-dire une quantité suffisante par rapport à la résine polymère pour que l'expansion de ladite résine se produise dans un laps de temps industriellement acceptable. Cette quantité efficace dépend bien entendu de la nature de la résine polymère à expanser et sa détermination dans chaque cas particulier est à la portée de l'homme de l'art en fonction des impératifs du processus industriel envisagé. Il est toutefois possible de préciser que le composé de formule (I) est rarement efficace pour l'expansion lorsqu'il est utilisé dans une proportion inférieure à 0,5 % en poids de la résine polymère. Il sera donc généralement utilisé donc une quantité au moins égale à 0,5 % du poids de la résine. Par ailleurs il est fréquent d'observer que la faculté d'expansion de la résine cesse d'augmenter lorsque la quantité de composé de formule (I) dépasse un certain niveau. Ici encore cette quantité maximale au-delà de laquelle l'efficacité de l'agent d'expansion selon l'invention cesse de croître dépend de la nature de la résine polymère, ainsi que de la nature des radicaux R¹, R, R³ et R⁴ et sa détermination pratique est à la portée de l'homme de l'art. Il est toutefois possible de préciser qu'il est généralement suffisant d'utiliser le composé de formule (I) dans une quantité au plus égale à 15 % en poids de la résine polymère.

La composition selon l'invention peut, outre la résine polymère et l'azoture d'hydrocarbone α, β hydroxylé, comprendre le cas échéant d'autres composants, notamment :
- un agent d'expansion classique tel qu'un composé azo, un hydrazide, un azide, un composé nitroso, la nitro urée, un carbonate ou bicarbonate ou un dérivé de guanyle, notamment lorsque son mélange avec l'azoture α, β hydroxylé permet de développer une synergie particulière dans la force d'expansion de la résine polymère ou une propriété particulière de la mousse synthétique obtenue.
- un agent tensio-actif adapté à la résine polymère. Ainsi dans le cas des résines phénol-aldéhyde il pourra s'agir des esters d'acides gras insaturés et de polyalkylène glycol, des condensats d'huile de ricin et d'oxyde d'alkylène, l'huile de ricin elle-même ou bien l'un de ses dérivés partiellement ou totalement hydrogénés et le cas échéant alkoxylés par exemple au moyen d'oxydes d'éthylène, de propylène ou de butylène, l'éthoxylate de nonylphénol, le copolymère polysiloxane-polyéthers et certains silicones modifiés.
- un catalyseur de réticulation, dans le cas de résines polymères thermodurcissables. Dans le cas de résines phénol-aldéhyde il pourra s'agir d'un catalyseur acide mminéral tel que l'acide sulfurique, l'acide phosphorique et leurs mélanges, ou bien d'un catalyseur acide organique tel que l'acide benzènesulfonique, l'acide p-toluène sulfonique, l'acide xylènesulfonique, l'acide cumènesulfonique, l'acide phénolsulfonique et leurs mélanges en toutes proportions. De tels catalyseurs acides sont généralement employés à raison de 4 % à 25 % environ du poids de la résine phénol-aldéhyde.
- un catalyseur de décomposition de l'azoture d'hydrocorbone α, β hydroxylé en une amine de formule (III). Il pourra s'agir notamment d'une phosphine telle qu'une triarylphosphine. Un tel catalyseur est généralement employé à raison de 1 % à 15 % environ en poids de la résine polymère.
- un composé minéral tel que notamment une zéolithe ou des fibres de verre.

Un second objet de la présente invention consiste en un procédé de fabrication de mousse synthétique consistant à soumettre à des conditions de moussage une composition comprenant une résine polymère et une quantité efficace, par rapport à ladite résine, d'au moins un agent d'expansion, caractérisé en ce que la composition soumise au moussage est une composition telle que décrite précédemment. Les conditions de moussage utilisables dans le procédé selon l'invention différent bien entendu selon l'agent d'hydrocarbone α, β hydroxylé particulier choisi d'une part et selon la nature de la résine polymère soumise à expansion d'autre part. Ces conditions ne différent toutefois pas considérablement des conditions d'expansion mises en oeuvre, pour une résine polymère donnée, avec les agents d'expansion traditionnelles. Le plus souvent ces conditions comprennent des conditions de température plus douces que celles pratiquées habituellement avec des agents d'expansion traditionnels. Dans le cas des résols phénoliques, par exemple, la composition est portée à une température comprise entre 20°C et 50°C environ pendant une durée - fonction de la température - comprise entre 1 et 10 minutes environ. Ces conditions de durée et de température permettent à la composition selon l'invention de s'expanser et de réticuler tout en remplissant la totalité du moule et en procurant des objets en mousse phénol-aldéhyde de bel aspect, possédant un coefficient d'isolation thermique satisfaisant. Le procédé selon l'invention permet d'obtenir sans difficulté des objets en mousse phénol-aldéhyde de densités très différentes, allant couramment de 40 à 150 kg/m³ environ. Lorsque des renforts tels que des fibres de verre sont ajoutés à la composition, on peut obtenir - selon la proportion de ces renforts dans la composition - des stratifiés allégés de densité allant de 150 à 800 kg/m³ environ.

Un dentier objet de la présente invention porte sur les produits finis en mousse synthétique obtenus par la mise en oeuvre du procédé décrit précédemment. Ces produits finis trouvent des applications très variées selon la nature de la résine polymère de départ. Dans le cas des résines phénol-aldéhyde on peut citer des plaques et panneaux de formes variées destinés à l'isolation thermique dans les industries du bâtiment, des travaux publics et des transports, par exemple pour la consolidation des galeries de mines et des tunnels, ainsi que des blocs à usage domestique tels que des blocs de mousse pique-fleurs. Dans le cas des polyuréthanes on peut citer des mousses qui, en raison de leur bonne résistance à la compression, sont utilisables dans l'ameublement comme garnitures de sièges et dans l'industrie automobile. Dans de cas des résines thermoplastiques telles que polychlorure de vinyle, polystyrène, polyéthyléne et polypropylène, on peut citer des mousses utilisées comme matériaux de calage et d'emballage. Les exemples ci-après sont fournis à titre illustratif et non limitatif de la présente invention. Sauf indication contraire, toutes les quantités sont exprimées en poids.

### Exemples 1 à 4

Une solution de 0,83 mole d'oxiranne dans 1300 ml de dioxanne est portée au reflux. On additionne alors goutte à goutte une solution de 1,15 mole de nitrure de sodium dans 175 ml d'eau. Le milieu réactionnel est maintenu au reflux pendant 20 heures. On récupère alors la phase organique et la phase aqueuse est extraite par 2 fois 100 ml de dioxanne. Les phases organiques sont regroupées, séchées sur sulfate de magnésium et le dioxanne est éliminé par distillation sous pression réduite. Le produit brut ainsi obtenu peut être utilisé sans purification ultérieure. On mélange, de manière à obtenir une solution limpide :
- 100 parties d'une résine formo-phénolique commercialisée par la société CRAY VALLEY SA sous la référence NORSOPHEN N 1205,
- X parties de surfactant (condensat d'oxyde d'éthylène et d'huile de ricin) commercialisé par la SOCIETE FRANCAISE D'ORGANOSYNTHESE sous la dénomination CEMUSOL B,
- Y parties de catalyseur commercialisé par la société CRAY VALLEY SA sous la dénomination C 2965 et constitué de 27,8 % en poids d'acide paratoluènesulfonique à 65 % de 64,8 % en poids d'acide phénolsulfonique à 70 %, de 3,7 % en poids de résorcinol et de 3,7 % en poids d'eau.
- Z parties d'un agent d'expansion préparé selon le mode de synthèse décrit ci-dessus. Cet agent d'expansion est l'azidophényléthanol (exemple 1), l'azidochloroproponol (exemple 2), l'azidobutanol (exemple 3) ou l'azidophénoxypropanol (exemple 4).

La solution obtenue est alors versée dans un moule à la température de 22°C. L'expansion se déclenche rapidement et atteint un facteur d'expansion (rapport du volume final au volume initial) V indiqué dans le tableau I ci-après en même temps que les valeurs de X, Y, et Z.

**TABLEAU I**

| Exemple | X | Y | Z | V |
|---|---|---|---|---|
| 1 | 4 | 13 | 5 | 8 |
| 2 | 4 | 13 | 2 | 10 |
| 3 | 3 | 13 | 5 | 12,5 |
| 4 | 3 | 15 | 5 | 8 |

### Exemples 5 à 7

On met en solution, dans 50 parties de polyéther polyol composant du polyuréthane commercialisé par la société WEBER S.A. sous la dénomination MARITHAN® EN, 5 parties d'un agent d'expansion préparé selon le mode de synthèse décrit aux exemples 1-4. L'agent d'expansion utilisé est l'azidophénoxypropanol (exemple 5), l'azidophényléthanol (exemple 6) ou l'azidochloropropanol (exemple 7). Par ailleurs on disperse dans 50 parties de diisocyanate de diphénylméthane W parties de triphénylphosphine. Les deux composants sont alors mélangés et le mélange versé dans un moule à la température de 22°C. L'expansion se déclenche rapidement et atteint un facteur d'expansion (rapport du volume final au volume initial) V indiqué dans le tableau II ci-après en même temps que la valeur de W.

| Exemple | 5 | 6 | 7 |
|---|---|---|---|
| W | 7 | 8 | 10 |
| V | 6 | 7 | 9 |

## Revendications

1. Composition capable d'être transformée en mousse comprenant un mélange à base de résine polymère et une quantité efficace, par rapport à ladite résine, d'agent d'expansion, ladite composition étant caractérisée en ce que l'agent d'expansion comprend au moins un azoture d'hydrocarbone α, β hydroxylé.

2. Composition selon la revendication 1, caractérisée en ce que l'agent d'expansion comprend au moins un composé de formule générale 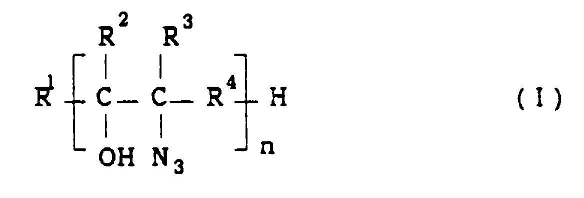 dans laquelle :
- R¹, R, R³, identiques ou différents, sont choisis parmi l'azoture d'hydrogène et les radicaux alkyle, aryle, aralkyle, alkylaryle et cycloalkyle ayant de préférence jusqu'à 20 atomes de carbone.
- R⁴ est choisi parmi les groupements alkylènes et arylènes ayant de préférence jusqu'à 20 atomes de carbone, et
- n est un nombre entier compris entre 1 et 20.

3. Composition selon l'une des revendications 1 et 2, caractérisée en ce que l'agent d'expansion comprend au moins un composé choisi parmi le 2-azido 1-phényl éthanol, le 1-azido 2-phényl éthanol, le 1-chloro 3- azido 2 propanol, le 3-chloro 2-azido propanol, le 1-azido 2-butanol, le 2-azido butanol, le 3-phénoxy 2-azido propanol, le 3 phénoxy 1-azido 2-propanol.

4. Composition selon l'une des revendications 1 à 3, caractérisée en ce que la résine est choisie parmi les polyuréthanes, le polystyrène, les copolymères styrène-anhydride maléique, le poly-isoprène, le polybutadiène, le polychlorure de vinyle, les copolymères chlorure de vinyle/acétate de vinyle, chlorure de vinyle/éthylène, chlorure de vinyle/chlorure de vinylidiène et chlorure de vinyle/acrylonitrile, le polyéthylène, le polypropylène, les copolymères tétrafluoroéthylène/hexafluoropropyléne, les copolymères éthylène/acétate de vinyle et éthylène/acrylate de butyle, les copolymères greffés de chlorure de vinyle et d'acrylate d'alkyle, les polyamides et les polylactames, ainsi que parmi les résines phénol-aldéhyde.

5. Composition selon l'une des revendications 1 à 4, caractérisée en ce que l'azoture d'hydrocarbone α, β hydroxylé est utilisé dans une quantité au moins égale à 0,5 % du poids de la résine polymére.

6. Composition selon l'une des revendications 1 à 5, caractérisée en ce que l'azoture d'hydrocarbone α, β hydroxylé est utilisé dans une quantité au plus égale à 15 % en poids de la résine polymère.

7. Composition selon l'une des revendications 1 à 6, caractérisée en ce qu'elle comprend en outre un agent d'expansion choisi parmi les composés azo, les hydrazides, les azides, les composés nitroso, la nitro urée, les carbonates et bicarbonates ou les dérivés de guanyle.

8. Composition selon l'une des revendications 1 à 7, caractérisée en ce qu'elle comprend en outre un agent tension-actif.

9. Composition selon la revendication 8, dans laquelle la résine polymère est une résine phénol-aldéhyde, caractérisée en ce que l'agent tensio-actif est choisi parmi les esters d'acides gras insaturés et de polyalkylène glycol, les condensats d'huile de ricin et d'oxyde d'alkylène, l'huile de ricin elle-même ou bien l'un de ses dérivés partiellement ou totalement hydrogénés et le cas échéant alkoxylés par exemple au moyen d'oxydes d'éthylène, de propylène ou de butylène, l'éthoxylate de nonylphénol, le copolymère polysiloxane-polyéthers et les silicones modifiés.

10. Composition selon l'une des revendications 1 à 8, dans laquelle la résine polymère est thermodurcissable, caractérisée en ce qu'elle comprend en outre un catalyseur de réticulation.

11. Composition selon la revendication 10, dans laquelle la résine polymère est une résine phénol-aldéhyde, caractérisée en ce que le catalyseur de réticulation est un acide minéral ou organique.

12. Composition selon la revendication 11, caractérisée en ce que ledit catalyseur est employé à raison de 4 % à 25 % du poids de la résine phénol-aldéhyde.

13. Composition selon l'une des revendications 2 à 12, caractérisée en ce qu'elle comprend en outre un catalyseur de décomposition de l'azoture d'hydrocarbone α, β hydroxylé en une amine de formule 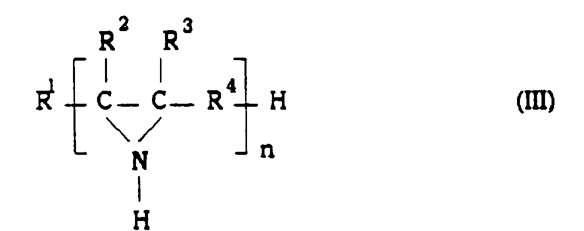 dans laquelle R¹, R, R³ et R⁴ ont les significations indiquées pour la formule (I) dans la revendication 2.

14. Composition selon la revendication 13, caractérisée en ce que ledit catalyseur de décomposition est une phosphine.

15. Composition selon l'une des revendications 13 et 14, caractérisée en ce que ledit catalyseur de décomposition est employé à raison de 1 % à 15 % en poids de la résine polymère.

16. Composition selon l'une des revendications 1 à 15, caractérisée en ce qu'elle comprend en outre un composé minéral choisi parmi les zéolithes et les fibres de verre.

17. Procédé de fabrication de mousse synthétique consistant à soumettre à des conditions de moussage une composition comprenant une résine polymère et une quantité efficace, par rapport à ladite résine, d'au moins un agent d'expansion, caractérisé en ce que la composition soumise au moussage est conforme à l'une des revendications 1 à 16.

18. Procédé de fabrication selon la revendication 17, caractérisé en ce que la résine polymère est un résol phénolique et en ce que la composition est portée à une température comprise entre 20°C et 50°C pendant une durée comprise entre 1 et 10 minutes.

19. Produits finis en mousse synthétique obtenus à partir d'une composition selon l'une des revendications 1 à 16.

## Patentansprüche

1. Foamable composition comprising a mixture based on polymer resin and an effective quantity, in relation to the said resin, of blowing agent, the said composition being characterised in that the blowing agent comprises at least one α,β-hydroxylated hydrocarbon azide.

2. Composition according to Claim 1, characterised in that the blowing agent comprises at least one compound of general formula 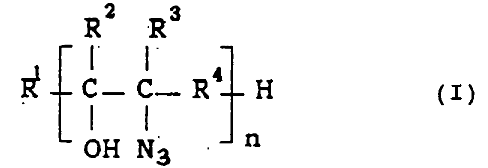 in which:
- R¹, R and R³, which are identical or different, are chosen from hydrogen azide and alkyl, aryl, aralkyl, alkylaryl and cycloalkyl radicals preferably containing up to 20 carbon atoms,
- R⁴ is chosen from alkylene and arylene groups preferably containing up to 20 carbon atoms, and
- n is an integer between 1 and 20.

3. Composition according to either of Claims 1 and 2, characterised in that the blowing agent comprises at least one compound chosen from 2-azido-1-phenylethanol, 1-azido-2-phenylethanol, 1-chloro-3-azido-2-propanol, 3-chloro-2-azidopropanol, 1-azido-2-butanol, 2-azidobutanol, 3-phenoxy-2-azidopropanol and 3-phenoxy-1-azido-2-propanol.

4. Composition according to one of Claims 1 to 3, characterised in that the resin is chosen from polyurethanes, polystyrene, styrene-maleic anhydride copolymers, polyisoprene, polybutadiene, polyvinyl chloride, vinyl chloride-vinyl acetate, vinyl chloride-ethylene, vinyl chloride-vinylidiene [sic] chloride and vinyl chloride-acrylonitrile copolymers, polyethylene, polypropylene, tetrafluoroethylene-hexafluoropropylene copolymers, ethylene-vinyl acetate and ethylene-butyl acrylate copolymers, graft copolymers of vinyl chloride and alkyl acrylate, polyamides and polylactams and from phenol-aldehyde resins.

5. Composition according to one of Claims 1 to 4, characterised in that the α,β-hydroxylated hydrocarbon azide is employed in a quantity of at least 0.5 % of the weight of the polymer resin.

6. Composition according to one of Claims 1 to 5, characterised in that the α,β-hydroxylated hydrocarbon azide is employed in a quantity of not more than 15 % by weight of the polymer resin.

7. Composition according to one of Claims 1 to 6, characterised in that it additionally includes a blowing agent chosen from azo compounds, hydrazides, azides, nitroso compounds, nitrourea, carbonates and bicarbonates or guanyl derivatives.

8. Composition according to one of Claims 1 to 7, characterised in that it additionally includes a surface-active agent.

9. Composition according to Claim 8, in which the polymer resin is a phenol-aldehyde resin, characterised in that the surface-active agent is chosen from polyalkylene glycol esters of unsaturated fatty acids, condensates of castor oil and alkylene oxide, castor oil itself or else one of its derivatives which are partially or completely hydrogenated and, if appropriate, alkoxylated, for example by means of ethylene, propylene or butylene oxides, nonylphenol ethoxylate, polysiloxane-polyethers copolymer and modified silicones.

10. Composition according to one of Claims 1 to 8, in which the polymer resin is heat-curable, characterised in that it additionally includes a crosslinking catalyst.

11. Composition according to Claim 10, in which the polymer resin is a phenol-aldehyde resin, characterised in that the crosslinking catalyst is an inorganic or organic acid.

12. Composition according to Claim 11, characterised in that the said catalyst is employed in a proportion of 4 % to 25 % of the weight of the phenol-aldehyde resin.

13. Composition according to one of Claims 2 to 12, characterised in that it additionally includes a catalyst for decomposition of the α,β-hydroxylated hydrocarbon azide to an amine of formula 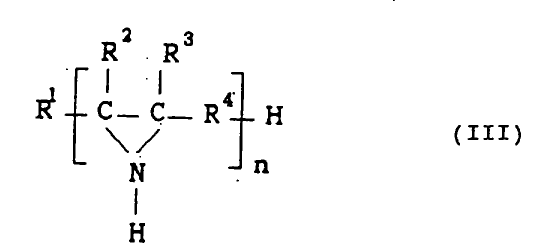 in which R¹, R, R³ and R⁴ have the meanings shown for formula (I) in claim 2.

14. Composition according to Claim 13, characterised in that the said decomposition catalyst is a phosphine.

15. Composition according to either of Claims 13 and 14, characterised in that the said decomposition catalyst is employed in a proportion of 1 % to 15 % by weight of the polymer resin.

16. Composition according to one of Claims 1 to 15, characterised in that it additionally includes an inorganic compound chosen from zeolites and glass fibres.

17. Process for the manufacture of synthetic foam, consisting in subjecting to foaming conditions a composition comprising a polymer resin and an effective quantity, in relation to the said resin, of at least one blowing agent, characterised in that the composition subjected to foaming is in accordance with one of Claims 1 to 16.

18. Process of manufacture according to Claim 17, characterised in that the polymer resin is a phenolic resole and in that the composition is taken to a temperature of between 20°C and 50°C for a period of between 1 and 10 minutes.

19. Finished products made of synthetic foam which are obtained by starting with a composition according to one of Claims 1 to 16.

## Claims

1. Aufschäumfähige Zusammensetzung, bestehend aus einer Polymerharz-Ausgangsmischung und einer im Verhältnis zum genannten Harz wirksamen Menge eines Aufschäummittels, wobei sich die genannte Zusammensetzung dadurch charakterisiert, daß das Aufschäumungsmittel mindestens ein hydroxyliertes a, b - Kohlenwasserstoffnitrid aufweist.

2. Zusammensetzung gemäß Patentanspruch 1, charakterisiert dadurch, daß das Aufschäumungsmittel mindestens eine Zusammensetzung folgender allgemeinen Formulierung beinhaltet: 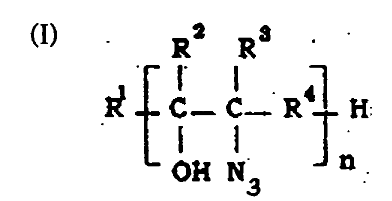 Gemäß dieser Formel werden **:**
- R¹, R und R³ in identischer oder unterschiedlicher Form aus dem Wasserstoffatom gewählt, wobei die Alkyl-, Aryl-, Aralkyl-, Alkylaryl und Cycloalkylradikale vorzugs-. weise bis zu 20 Kohlenstoffatome aufweisen,
- R⁴ wird aus den Alkylen- und Arylengruppierungen ausgewählt, die vorzugsweise bis zu 20 Kohlenstoffatome besitzen,
- n stellt hier eine Ganzzahl zwischen 1 und 20 etwa dar.

3. Zusammensetzung gemäß einer der Patentforderungen 1 und 2, charakterisiert dadurch, daß das Aufschäumungsmittel mindestens eine Verbindung folgender Ketten aufweist:
2-Azido-1-Phenyläthanol/ 1-Azido-2-Phenyläthanol/ 1-Chloro-3-Azido-2-Propanol/3-Chloro-2-Azido-Propanol/ 1-Azido-2-Butanol/ 2-Azido-Butanol/ 3-Phenoxy-2-Azidopropanol/ 3-Phenoxy-1-Azido-2-Propanol.

4. Zusammensetzung gemäß einer der Patentansprüche 1 bis 3, dadurch charakterisiert, daß als Harz eine folgender Ketten Verwendung findet: Polyurethane, Polystyrol, Malein-Anhydridstyrol-Copolymere, Polyisopren, Butadien, Polyvinylchlorid, Mischpolymerisate aus Vinylchlorid/Vinylacetat,Vinylchlorid/Äthylen, Vinylchhlorid/Vynilidenchlorid und Vynilchlorid/Acrylnitril, Polyäthylen, Polypropylen,Mischpolymerisate aus Tetrafluoräthylen/Hexafluoropropylen, Mischpolymerisate aus Äthylen/Vynilacetat und Äthylen/Butylacrylat, Propfmischpolymerisate aus Vinylchlorid und Alkylacrylat, Polyamide und Polyaktame sowie ein Harz aus der Gruppe der Aldehydphenolharze

5. Zusammensetzung gemäß einer der Patentansprüche 1 bis 4, dadurch charakterisiert, daß das hydroxylierte α, β-Kohlenwasserstoffnitrid eingesetzt wird in einer Menge gleich 0,5 Gewichtsprozent des Polymerharzes.

6. Zusammensetzung gemäß einer der Patentansprüche bis 5, dadurch charakterisiert, daß
das hydroxylierte α, β-Kohlenwasserstoffnitrid eingesetzt wird in einer Menge gleich 15 Gewichtsprozent des Polymerharzes.

7. Zusammensetzung gemäß einer der Patentansprüche 1 bis 6, dadurch charakterisiert, daß diese außerdem ein Aufschäumungsmittel enthält, ausgewählt aus den Verbindungen der Azo-Gruppen, Hydrazide, Azide, Nitroso-Verkettungen, Nitro-Urea, Carbonaten und Bicarbonaten oder Guanylderivaten.

8. Zusammensetzung gemäß einer der Patentansprüche 1 bis 7, dadurch charakteri-siert, daß diese im weiteren einen oberflächenaktiven Stoff enthält.

9. Zusammensetzung gemäß Patentspruch 8, nach der das Polymer ein Aldehydphenolharz ist, charakterisiert dadurch, daß die grenzflächenaktive Substanz ausgewählt wird aus den ungesättigten Fettsäureestern sowie dem Glykolpolyalkylen, aus Rizinusölkondensaten und Alkylenoxyd oder Rizinusöl selbst oder einem seiner Derivate in teilweise oder vollständiger hydrierter Form oder ggf. alkoxyliert, z.B. mithilfe von Äthylen-, Propylen- oder Butyulenoxyden, Nonylphenoläthoxylat, Polyätherpolysiloxan-Copolymeren oder auch modifizierter Silikone.

10. Zusammensetzung gemäß einer der Patentansprüche 1 bis 8, in der das Polymerharz warmhärtend ist, charakterisiert dadurch, daß es unter anderem einen Vernetzungskatalysator enthält.

11. Zusammensetzung gemäß Patentanspruch 10, in der das Polymerharz ein Phenolaldehydharz darstellt, charakterisiert dadurch, daß der Vernetzungskatalysator eine mineralische oder organische Säure ist.

12. Zusammensetzung gemäß Patentanspruch 11, dadurch charakterisiert, daß der genannte Katalysator eingesetzt wird mit einem Anteil zu 4 bis 25 Gewichtsprozent des Phenolaldehydharzes.

13. Zusammensetzung gemäß einer der Patentansprüche 2 bis 12, dadurch charakterisiert, daß diese im weiteren einen Zerfallskatalysator aufweist, der durch hydroxyliertes α, β - Kohlenwasserstoffnitrid als Amin folgender Formulierung enthalten ist: 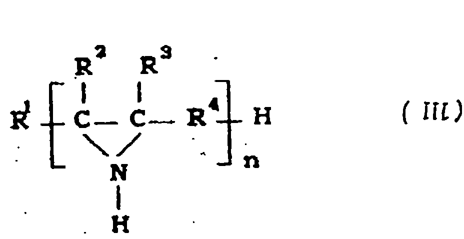 In dieser Verbindung weisen R¹, R, R³ und R⁴ die für die Formel angegebene Bedeutung auf (I) im Patentanspruch 2.

14. Zusammensetzung gemäß Patentanspruch 13, dadurch charakterisiert, daß der genannte Zerfallskatalysator ein Phosphin ist.

15. Zusammensetzung gemäß einer der Patentansprüche 13 und 14, dadurch charakterisiert, daß der genannte Zerfallskatalysator eingesetzt wird zu 1 bis 15 Gewichtsprozent des Polymerharzes.

16. Zusammensetzung gemäß einer der Patentansprüche 1 bis 15, dadurch charakterisiert, daß diese im weiteren eine mineralische Verbindung aus der Zeolithgruppe und zusätzlich Glasfasern enthält.

17. Herstellverfahren für Kunststoffschaum, bestehend daraus, eine Verbindung Aufschläumungsbedingungen zu unterziehen, wobei diese ein polymeres Harz und im Verhältnis zu diesem Harz eine wirksame Menge von mindestens einem Aufschäummittel enthält, dadurch charakterisiert, daß diese aufzuschäumende Zusammensetzung den Angaben der Patentansprüche 1 bis 16 entspricht.

18. Herstellverfahren gemäß Patentanspruch 17, dadurch charakterisiert, daß das Polymerharz ein Phenolresolharz ist und die Zusammensetzung auf eine Temperatur zwischen 20°C und 50°C über eine Dauer von 1 bis 10 Minuten gebracht wird.

19. Kunststoffschaumstoff-Fertigprodukte, erzielt auf der Grundlage lt. einer der Patentansprüche 1 bis 16 genannten Zusammensetzung.
